(19) Europäisches Patentamt
European Patent Office
Office européen des brevets

(11) **EP 4 265 277 A1**

(12) # EUROPEAN PATENT APPLICATION
published in accordance with Art. 153(4) EPC

(43) Date of publication:
**25.10.2023 Bulletin 2023/43**

(21) Application number: **21907049.7**

(22) Date of filing: **14.12.2021**

(51) International Patent Classification (IPC):
*A61K 48/00* (2003.01)     *A61K 47/54* (2017.01)
*A61K 47/64* (2017.01)     *A61K 31/7088* (2006.01)
*A61P 35/00* (2006.01)     *C12N 15/113* (2010.01)

(52) Cooperative Patent Classification (CPC):
**A61K 31/7088; A61K 47/54; A61K 47/64;
A61K 48/00; A61P 35/00; C12N 15/113**

(86) International application number:
**PCT/KR2021/018965**

(87) International publication number:
**WO 2022/131745 (23.06.2022 Gazette 2022/25)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority: **15.12.2020 KR 20200175271**

(71) Applicant: **Seasun Therapeutics
Daejeon 34015 (KR)**

(72) Inventors:
• **PARK, Min-Jung
Daejeon 35064 (KR)**
• **KIM, Hye Joo
Daejeon 34080 (KR)**
• **YU, Ji-Yeon
Cheongju-si, Chungcheongbuk-do 28193 (KR)**
• **PARK, Hee Kyung
Daejeon 34034 (KR)**

(74) Representative: **Mewburn Ellis LLP
Aurora Building
Counterslip
Bristol BS1 6BX (GB)**

Remarks:
The complete document including Reference
Table(s) and the Sequence Listing(s) can be
downloaded from the EPO website

(54) ## COMPOSITION FOR PREVENTING OR TREATING GLIOBLASTOMA COMPRISING PEPTIDE NUCLEIC ACID COMPLEX AS ACTIVE INGREDIENT

(57) The present invention relates to a pharmaceutical composition, for treating glioblastoma, comprising a nucleic acid complex as an active ingredient and, more particularly, to a pharmaceutical composition, for preventing or treating glioblastoma, comprising a nucleic acid complex having complementarily bound: a bioactive peptide nucleic acid having a sequence binding to TGF-β2 gene; and a carrier peptide nucleic acid. The nucleic acid complex according to the present invention has excellent cell permeability and intracellular activity and can effectively inhibit the expression of TGF-β2 gene and the hypostatic gene thereof, and thus is useful in preventing or treating glioblastoma.

EP 4 265 277 A1

**(Cont. next page)**

FIG. 4

NC : Negative control
PC : Positive control
(galunisertib)

**Description**

**Technical Field**

The present invention relates to a pharmaceutical composition for preventing or treating glioblastoma comprising a nucleic acid complex as an active ingredient and more particularly, to a pharmaceutical composition for preventing or treating glioblastoma comprising a nucleic acid complex in which a carrier peptide nucleic acid complementarily binds to a bioactive peptide nucleic acid having a sequence capable of binding to a TGF-β2 gene.

**Background Art**

Glioblastoma is a tumor with the highest degree of malignancy, in which nuclear atypia, mitotic phase, proliferation of vascular endothelial cells and necrosis are histologically observed, among tumors arising from glial cells in the brain. Glioblastoma accounts for the majority of gliomas, is the second most histologically frequent, and is the most frequent malignant tumor in the brain and central nervous system with a 5-year survival rate of only 5% (USA). In Korea, glioblastoma is the most common encephalotheloma that accounts for 15.1% of all brain and CNS protocarcinomas and accounts for 34.4% of all gliomas.

Glioblastoma is a very fast-growing tumor and as a result, intracranial pressure rises rapidly, causing headache, nausea, vomiting, or the like, and frequent convulsions in adults. In addition, the tumor itself or cerebral edema accompanying the tumor causes deterioration in the functions of nearby nerves and results in various symptoms such as limb movement loss, sensory deterioration, facial paralysis, language disorder, cognitive decline, left-right discrimination disorder and the like. Neurological deficits in these parts of the body are very diverse depending on the location of the tumor, so it is impossible to diagnose based on only one symptom.

When glioblastoma occurs, the tumor is usually excised as much as possible through surgical removal. The extent of resection should be carefully determined because glioblastoma removal may cause serious complications. Radiation therapy and chemotherapy are performed after glioblastoma is confirmed histologically through removed tissue after surgery.

The standard first-line treatment for glioblastoma is a combination therapy of surgical therapy, chemotherapy such as temozolomide (TMZ) and radiation therapy (RT).

The NCCN guidelines for central nervous system cancer showed that TMZ, which is an alkylating (methylating) agent, is currently known as a standard therapy along with postoperative RT for GBM patients with good prognosis at a young age. However, the patient group to which this standard therapy can be applied is very limited and the prognosis after treatment is so poor that the 5-year survival rate of all glioblastoma patients is less than 5%.

In addition, despite therapy including surgery, radiation therapy and chemotherapy, glioblastoma retains stem cell-like cancer cells called "glioblastoma stem cells" and thus has very high treatment resistance, resulting in poor prognosis after treatment.

According to this need, research to develop a novel therapeutic agent for glioblastoma is actively conducted. For example, International Patent Publication No. 2012-061120 discloses a method for treating glioblastoma using purified glioblastoma GBM6-AD stem cells, and International Patent Publication No. 2012-104822 discloses a method of treating glioblastoma by administration of Macitentan.

TGF-β2 is known as an oncogene that regulates the tumor microenvironment through the Smad downstream pathway and is essential for tumor proliferation, migration, and invasion (Joanna L. Birch et al., Cellular Signaling, 2020, Volume 72, 109638). In particular, TGF-β2 was highly expressed in glioblastoma patients and it was found that the patient's survival rate correlates with gene expression rate (Roy LO, Poirier MB, Fortin D., Int. J. Mol. Sci., 2018, 19(4), 1113).

Meanwhile, unlike traditional drugs, nucleic acid drugs inhibit the expression of target-specific messenger RNA (mRNA) and thus research is ongoing into the use of nucleic acid drugs in areas in which treatment with conventional drugs that target proteins is impossible (Kole R. et al., Nature Rev. Drug Discov. 11;125, 2012., Wilson C. et al., Curr. Opin. Chem. Bio. 2006; 10:607, 2006). Various clinical trials using nucleic acids are in progress due to performance and advantages thereof as drugs and the use of carriers for intracellular introduction or blood-brain barrier penetration is extremely limited despite the increasing use of nucleic acid-based therapeutics.

Recently, in order to promote safer and more effective use of drugs, not only formulations suitable for drugs but also methods of efficiently delivering drugs to target sites in the body are receiving attention. In particular, there is demand for practical application of a drug delivery system (DDS) that efficiently transports a drug in a required amount to a required place when needed.

In this regard, the present inventors have found that a nucleic acid complex in which a bioactive nucleic acid complementarily binds to a carrier peptide nucleic acid modified to have a net positive charge has surprisingly improved cell permeability, and this enables the expression of the target gene to be regulated very efficiently, and filed a patent application on a novel construct with low cytotoxicity and improved cell permeability and ability to regulate gene expression of bioactive nucleic acids (PCT/KR2017/008636). In addition, the present inventors have continuously conducted research on the function of the construct and developed a novel construct having the ability to penetrate the blood-brain barrier with excellent efficiency (Korean Patent Publication No. 10-2019-0128465).

Accordingly, as a result of intensive efforts to apply nucleic acid complexes having excellent cell permeability to the treatment of glioblastoma, the present inventors have found that a nucleic acid complex in which a bioactive peptide nucleic acid targeting the TGF-β2 gene complementarily binds to a carrier peptide nucleic acid is highly effective in preventing or treating glioblastoma and thus completed the present invention.

**Summary of the Invention**

It is an object of the present invention to provide a pharmaceutical composition for preventing or treating glioblastoma comprising a nucleic acid complex as an active ingredient.

In order to accomplish the above objects, the present invention provides a pharmaceutical composition for preventing, ameliorating or treating glioblastoma comprising, as an active ingredient, a cell-permeable nucleic acid complex in which a carrier peptide nucleic acid complementarily binds to a bioactive nucleic acid targeting a TGF-β2 gene.

In addition, the present invention provides a method of treating or preventing glioblastoma comprising administering a cell-permeable nucleic acid complex in which a carrier peptide nucleic acid complementarily binds to a bioactive nucleic acid targeting a TGF-β2 gene.

In addition, the present invention provides the use of the cell-permeable nucleic acid complex in which a carrier peptide nucleic acid complementarily binds to a bioactive nucleic acid targeting a TGF-β2 gene for the prevention or treatment of glioblastoma.

In addition, the present invention provides the use of the cell-permeable nucleic acid complex in which a carrier peptide nucleic acid complementarily binds to a bioactive nucleic acid targeting a TGF-β2 gene for the manufacture of a medicament for preventing or treating glioblastoma.

**Brief Description of Drawings**

FIG. 1 shows the expression of TGF-β target genes and downstream genes for each combination after treating human glioblastoma cells (U87MG) with the peptide nucleic acid complex of the present invention, more particularly, FIG. 1A shows results of combinations 1 to 5 and FIG. 1B shows the results of combinations 1 and 6.

FIG. 2A shows the cell migration for each combination after treating human glioblastoma cells (U87MG) with the peptide-nucleic acid complex of the present invention and FIG. 2B shows the results of quantification thereof.

FIG. 3 shows the phenotype of the tumor tissue obtained after treating an orthotopic animal model of human glioblastoma cells (U87MG) with the peptide-nucleic acid complex of the present invention.

FIG. 4 shows the phenotype of the obtained tumor tissue after treating an orthotopic animal model of human glioblastoma cells (U87MG) with the peptide-nucleic acid complex of the present invention by concentration.

FIG. 5 shows the expression of TGF-β-targeting genes and downstream genes in the tumor tissue obtained after treating an orthotopic animal model of human glioblastoma cells (U87MG) with the peptide nucleic acid complex of the present invention, more particularly, FIG. 5A shows results of combination 3 and FIG. 5B shows the results of combination 6.

**Detailed Description and Preferred Embodiments of the Invention**

Unless defined otherwise, all technical and scientific terms used herein have the same meanings as appreciated by those skilled in the field to which the present invention pertains. In general, the nomenclature used herein is well-known in the art and is ordinarily used.

The present inventors aimed to determine whether or not administration of a nucleic acid complex in which a bioactive peptide nucleic acid targeting a TGF-β2 gene complementarily binds to a carrier peptide nucleic acid is highly effective in preventing or treating glioblastoma.

That is, in one embodiment of the present invention, administration, to glioblastoma cell lines and glioblastoma cell line orthotopic models, of a nucleic acid complex, in which a bioactive peptide nucleic acid having a sequence binding to a TGF-β2 gene complementarily binds to a carrier peptide nucleic acid, causes efficient inhibition of the expression of the TGF-β2 genes and downstream genes thereof and thus effectively treats glioblastoma.

Accordingly, in one aspect, the present invention is directed to a pharmaceutical composition for preventing, ameliorating or treating glioblastoma comprising, as an active ingredient, a nucleic acid complex in which a carrier peptide nucleic acid complementarily binds to a bioactive nucleic acid targeting a TGF-β2 gene.

In the present invention, the bioactive nucleic acid may be bioactive peptide nucleic acid.

In the present invention, the nucleic acid complex in which the bioactive peptide nucleic acid complementarily binds to the carrier peptide has a structure represented by the following Formula (1):

$$\text{Formula (1)}$$

$$[A \equiv C^{(+)}]$$

Wherein

A is a bioactive nucleic acid having a sequence capable of binding to a target gene or a target gene sequence,
C is a carrier peptide nucleic acid capable of binding to the bioactive nucleic acid, and
'≡' means complementary binding between the bioactive nucleic acid and the carrier peptide nucleic acid,
wherein the bioactive nucleic acid represented by A is a peptide nucleic acid which has a net negative charge,
the carrier peptide nucleic acid represented by $C^{(+)}$ has a net positive charge,
the nucleic acid complex of Formula (1) represented by $A \equiv C^{(+)}$ has a net positive charge, and
the carrier peptide nucleic acid comprises at least one gamma- or alpha-backbone-modified peptide nucleic acid monomer such that the carrier peptide nucleic acid has an net positive charge, and the gamma- or alpha-backbone-modified peptide nucleic acid monomer comprises monomers having positively charged amino acids, the number of which is higher than the number of monomers having negatively charged amino acids, so the carrier peptide nucleic acid has a net positive charge.

As used herein, the term "bioactive nucleic acid" refers to a nucleic acid having a complementary sequence capable of binding to a gene that is the target of expression inhibition, in particular, a nucleic acid having a complementary sequence capable of binding to the mRNA of the gene that is the target of expression inhibition, or a nucleic acid having a sequence that is the target of the expression promotion, means a nucleic acid involved in the regulation of gene expression, such as inhibiting or promoting the expression of the corresponding gene, or a nucleic acid having a sequence complementary to a gene that is the target of expression inhibition or promotion, or a nucleic acid having a sequence complementary to a single-stranded RNA sequence such as pre-mRNA, miRNA, and mRNA.

In particular, the "bioactive peptide nucleic acid" used herein binds to a target gene and a nucleotide sequence comprising the same *in vitro* or *in vivo* and thus acts to activate or inhibit the inherent functions (transcript expression or protein expression) of the corresponding gene, or to regulate splicing of pre-mRNA (e.g., exon skipping), and the base sequence is a gene regulatory sequence, gene coding sequence or a splicing regulatory sequence. The gene regulatory region is a promoter, a transcriptional enhancer, a 5' untranslated region, a 3' untranslated region, a viral packaging sequence, and a selectable marker. The gene region may be an exon or an intron, and the gene region may be present within 10, 5, 3, or 1 kb or 500, 300, or 200 bp of the transcription initiation site of the gene, for example, upstream or downstream of the initiation site. In addition, the splicing regulatory region may comprise sequences related to exon skipping, cryptic splicing, pseudo-splice site activation, intron retention, and alternative splicing deregulation.

Therefore, the "bioactive nucleic acid" in the present invention is preferably an antisense peptide nucleic acid of TGF-β2, which is a target gene for glioblastoma, and has more preferably a sequence represented by SEQ ID NO: 1, but is not limited thereto.

As used herein, the term "carrier peptide nucleic acid" refers to a nucleic acid, the bases of which are partly or entirely complementarily bound to the bioactive nucleic acid, to impart functionality thereto, and the carrier peptide nucleic acid used herein may be a peptide nucleic acid (PNA) or a modified nucleic acid similar thereto, and is preferably a peptide nucleic acid, but is not limited thereto.

In particular, the carrier peptide nucleic acid preferably comprises a sequence selected from the group consisting of sequences represented by SEQ ID NOS: 4 to 7 and 9, but is not limited thereto.

As used herein, the nucleic acid complex is capable of allowing the bioactive substance to enter the body, ultimately cells, and specifically is capable of delivering the bioactive substance into the body through the blood-brain barrier.

Accordingly, in the present invention, the nucleic acid complex may be capable of penetrating the blood-brain barrier.

In the present invention, each of the bioactive nucleic acid and the carrier peptide nucleic acid may comprise 2 to 50, preferably 5 to 30, more preferably 10 to 25, most preferably 15 to 17 nucleic acid monomers.

In the present invention, the nucleic acid complex comprises a bioactive nucleic acid having a sequence represented by SEQ ID NO: 1, and a carrier peptide nucleic acid having a sequence represented by any one selected from the group consisting of SEQ ID NOS: 4 to 7 and 9, but is not limited thereto.

The composition of the present invention comprises, as an active ingredient, (i) a nucleic acid complex comprising a bioactive nucleic acid having a sequence represented by SEQ ID NO: 1 and a carrier peptide nucleic acid having a sequence represented by SEQ ID NO: 4;

(ii) a nucleic acid complex comprising a bioactive nucleic acid having a sequence represented by SEQ ID NO: 1 and a carrier peptide nucleic acid having a sequence represented by SEQ ID NO: 5;

(iii) a nucleic acid complex comprising a bioactive nucleic acid having a sequence represented by SEQ ID NO: 1 and a carrier peptide nucleic acid having a sequence represented by SEQ ID NO: 6;

(iv) a nucleic acid complex comprising a bioactive nucleic acid having a sequence represented by SEQ ID NO: 1 and a carrier peptide nucleic acid having a sequence represented by SEQ ID NO: 7; and

(v) a nucleic acid complex comprising a bioactive nucleic acid having a sequence represented by SEQ ID NO: 1 and a carrier peptide nucleic acid having a sequence represented by SEQ ID NO: 9.

I

n the present invention, the bioactive nucleic acid or the carrier peptide nucleic acid may further comprise a material for facilitating endosomal escape that is additionally bound to the 5'-end or 3'-end of each nucleic acid. That is, the bioactive nucleic acid or the carrier peptide nucleic acid may further comprise a material for facilitating endosomal escape of the bioactive nucleic acid and the carrier peptide nucleic acid and thus have the structure represented by the following Formula (2).

$$\text{Formula (2)}$$

$$[mA \equiv mC^{(+)}]$$

Wherein

"m" represents a material for facilitating endosomal escape of the bioactive nucleic acid and the carrier peptide nucleic acid.

In the present invention, the "material for facilitating endosomal escape" may facilitate escape of the bioactive nucleic acid from the endosomes by increasing the osmotic pressure in the endosomes or destabilizing the membrane of the endosomes, which means that the "material that facilitates endosomal escape" enables the bioactive nucleic acid to move more efficiently and rapidly to the nucleus or cytoplasm and to meet and function with the target gene (D. W. Pack, A. S. Hoffman, S. Pun, P. S. Stayton, "Design and development of polymers for gene delivery," Nat. Rev. Drug. Discov., 4, 581-593 (2005)).

In the present invention, the material for facilitating endosomal escape may be at least one selected from the group consisting of peptides, lipid nanoparticles, polyplex nanoparticles, polymer nanospheres, inorganic nanoparticles, cationic lipid-based nanoparticles, cationic polymers, and pH-sensitive polymers.

In the present invention, as the material for facilitating endosomal escape, a peptide having a sequence of GLFDIIKKIAESF (SEQ ID NO: 10) may be bound to the bioactive nucleic acid via a linker, and histidine(10) may be bound to the carrier peptide nucleic acid via a linker, but the present invention is not limited thereto.

In the present invention, the lipid nanoparticles may be selected from the group consisting of lipids, phospholipids, acetyl palmitate, poloxamer 18, Tween 85, tristearin glyceride, and Tween 80.

In the present invention, the polyplex nanoparticles may be poly(amidoamine) or polyethylenimine (PEI).

In the present invention, the polymer nanospheres may be selected from the group consisting of polycaprolactone, poly(lactide-co-glycolide), polylactide, polyglycolide, poly(d,l-lactide), chitosan, and PLGA-polyethylene glycol.

In the present invention, the inorganic nanoparticles may be selected from the group consisting of $Fe_2O_3$, $Fe_3O_4$, $WO_3$ and $WO_{2.9}$.

In the present invention, the cationic lipid-based nanoparticles may be selected from the group consisting of 1-(aminoethyl)iminobis[N-(oleicylcysteinyl-1-aminoethyl)propionamide], an N-alkylated derivative of PTA, and 3,5-didodecyloxybenzamidine.

In the present invention, the cationic polymer may be selected from the group consisting of vinylpyrrolidone-N,N-dimethylaminoethyl methacrylate acid copolymer diethyl sulfate, polyisobutylene, and poly(N-vinylcarbazole).

In the present invention, the pH-sensitive polymers may be selected from the group consisting of polyacids, poly(acrylic acid), poly(methacrylic acid) and hydrolyzed polyacrylamide.

In the present invention, the bioactive nucleic acid may consist of a natural nucleic acid base and/or a modified nucleic acid monomer. The carrier peptide nucleic acid may have a sequence partially or entirely complementary to the base sequence of the bioactive nucleic acid.

In particular, the carrier peptide nucleic acid may comprise one or more universal bases and all of the carrier peptide nucleic acids may comprise universal bases.

In the present invention, the bioactive nucleic acid is selected from the group consisting of DNA, RNA, or modified nucleic acids including peptide nucleic acid (PNA), phosphorodiamidate morpholino oligonucleotide (PMO), locked nucleic acid (LNA), glycol nucleic acid (GNA), and threose nucleic acid (TNA), antisense oligonucleotide, aptamers, small interfering RNA (siRNA), short hairpin RNA (shRNA), ribozyme, and DNAzyme. Preferably, the bioactive nucleic acid is selected from the group consisting of DNA, RNA, or modified nucleic acids including peptide nucleic acid (PNA), phosphorodiamidate morpholino oligonucleotide (PMO), locked nucleic acid (LNA), glycol nucleic acid (GNA), and threose nucleic acid (TNA).

In the present invention, when the monomer used for the bioactive nucleic acid is PNA, it is called a bioactive peptide nucleic acid, and when other monomers are used, they are referred to in the same manner.

In the present invention, the bioactive nucleic acid and the carrier peptide nucleic acid may further comprise at least one functional group selected from the group consisting of phosphodiester, 2'-0-methyl, 2'-methoxy-ethyl, phosphoramidate, methylphosphonate, and phosphorothioate.

In the present invention, the carrier peptide nucleic acid may have a nucleotide sequence which is partially or entirely complementary to the bioactive nucleic acid. In particular, the carrier peptide nucleic acid may comprise at least one universal base, and the carrier peptide nucleic acid may be entirely composed of universal bases.

In the present invention, with regard to the electrical properties, each of the bioactive nucleic acid and the carrier peptide nucleic acid in the nucleic acid complex may have a net positive charge (positive), a net negative charge (negative), or neutral (no charge).

The term "net" in the expression of the electrical properties means overall electrical properties of respective charges of the bioactive nucleic acids or carrier peptide nucleic acids as viewed from the outside, not the electrical properties of individual bases. For example, even though some monomers in the bioactive nucleic acid are positive, when the number of negatively charged monomers is greater than the number of positively charged monomers, the bioactive nucleic acid is negatively charged in terms of the "net" electrical properties. When the number of positively charged bases and/or backbones is greater than the number of negatively charged bases and/or backbones, even though some bases and/or backbone constituents in the carrier peptide nucleic acid are negatively charged, the carrier peptide nucleic acid is considered to be positively charged in terms of the "net" electrical properties thereof.

Accordingly, the nucleic acid complex of the present invention may be considered to have a net positive charge. In the nucleic acid complex, preferably, the bioactive nucleic acid has a net negative charge or is neutral in terms of overall electrical properties, and the carrier peptide nucleic acid has a net positive charge in terms of overall electrical properties, but is not limited thereto.

In the present invention, a modified peptide nucleic acid monomer may be used to impart electrical properties to the bioactive nucleic acid and the carrier peptide nucleic acid, and the modified peptide nucleic acid monomer comprises, as a positively charged carrier peptide nucleic acid, at least one positively charged nucleic acid selected from the group consisting of lysine (Lys, K), arginine (Arg, R), histidine (His, H), diamino butyric acid (DAB), ornithine (Orn), and amino acid analogs, and comprises, as a negatively charged carrier peptide nucleic acid, glutamic acid (Glu, E), which is a negatively charged amino acid, or an amino acid analogue, which is a negatively charged amino acid.

In the present invention, the carrier peptide nucleic acid may comprise at least one gamma- or alpha-backbone-modified peptide nucleic acid monomer in order for the carrier peptide nucleic acid to have a net positive charge.

The gamma- or alpha-backbone-modified peptide nucleic acid monomer comprises, in the backbone thereof, at least one positively charged amino acid selected from the group consisting of lysine (Lys, K), arginine (Arg, R), histidine (His, H), diamino butyric acid, ornithine (Orn), and amino acid analogs in order for the carrier peptide nucleic acid to have a net positive charge.

In the present invention, the peptide nucleic acid monomer having a modified nucleobase, in addition to the backbone modification, for modification of the peptide nucleic acid monomer so as to impart an electrical charge thereto may be used. Preferably, an amine, triazole or imidazole moiety may be comprised in the nucleobase to impart a positive charge thereto, or carboxylic acid may be comprised in the base to impart a negative charge thereto.

In the present invention, the modified peptide nucleic acid monomer of the carrier peptide nucleic acid may further comprise a negative charge in the backbone or nucleobase. However, preferably, the modified peptide nucleic acid monomer comprises more positively charged monomers than negatively charged monomers, such that the carrier peptide nucleic acid is positively charged.

In the nucleic acid complex according to the present invention, at least one material selected from the group consisting of a hydrophobic moiety, a hydrophilic moiety, a target-antigen-specific antibody, an aptamer, and a fluorescent/luminescent marker is bound to the bioactive nucleic acid and/or the carrier peptide nucleic acid. Preferably, at least one material selected from the group consisting of a hydrophobic moiety, a hydrophilic moiety, a target-antigen-specific antibody, an aptamer and a fluorescent/luminescent marker for imaging may be bound to the carrier peptide nucleic acid.

In the present invention, the binding of at least one material selected from the group consisting of a hydrophobic moiety, a hydrophilic moiety, a target-antigen-specific antibody, an aptamer, a quencher, a fluorescent marker and a luminescent marker to the bioactive nucleic acid and/or the carrier peptide nucleic acid may be a simple covalent bond or a covalent bond mediated by a linker, but is not limited thereto. Preferably, cell permeation, solubility, stability, transportation and imaging-related substances (e.g., hydrophobic residues and the like) bound to the nucleic acid carrier exist independently of the bioactive nucleic acid that regulates the expression of the target gene.

In the present invention, as described above, the complementary binding of the bioactive nucleic acid and the carrier peptide nucleic acid is generally antiparallel binding or parallel binding. Complementary binding forms a structure that is separated in the presence of the target sequence of the bioactive nucleic acid (a sequence complementary to the bioactive nucleic acid).

The antiparallel binding and parallel binding are determined depending on the 5'-direction and the 3'-direction in the binding mode of DNA-DNA or DNA-PNA. Antiparallel binding is a general binding mode of DNA-DNA or DNA-PNA. For example, in the nucleic acid complex according to the present invention, the bioactive nucleic acid in a 5' to 3' direction is bound to the carrier peptide nucleic acid in a 3' to 5' direction. Parallel binding has slightly lower binding force than antiparallel binding, and the bioactive nucleic acid and the carrier peptide nucleic acid are bound to each other in the same direction, that is, the 5' to 3' direction or the 3' to 5' direction.

In the nucleic acid complex according to the present invention, preferably, the binding force between the bioactive nucleic acid and the carrier peptide nucleic acid is lower than the binding force between the bioactive nucleic acid and the target gene targeted by the bioactive nucleic acid, particularly the mRNA of the target gene. The bonding force is determined by the melting temperature (Tm).

Examples of specific methods for lowering the binding force (melting temperature, Tm) between the bioactive nucleic acid and the carrier peptide nucleic acid, compared to the binding force between the bioactive nucleic acid and the target gene targeted by the bioactive nucleic acid, particularly the mRNA of the target gene, comprise parallel binding or partial specific binding between the bioactive nucleic acid and the carrier peptide nucleic acid, but are not limited thereto.

As another example, the carrier peptide nucleic acid comprises at least one peptide nucleic acid base selected from the group consisting of a linker, a universal base, and a peptide nucleic acid base comprising a base that is not complementary to a corresponding base of the bioactive nucleic acid, but is not limited thereto.

In the present invention, the universal base is a base that is non-selectively bound to a natural base such as adenine, guanine, cytosine, thymine or uracil, and has a binding force lower than the complementary binding force, and may be at least one selected from the group consisting of inosine PNA, indole PNA, nitroindole PNA, and abasic PNA, and is preferably inosine PNA.

The present invention provides a combination of the binding mode and electrical properties of nucleic acids for controlling the function of the nucleic acid complex, and controls the particle size and the action time using the combination of the binding mode and electrical properties of the nucleic acids, and improves cell permeability, solubility and specificity.

In the present invention, the time point at which the bioactive peptide nucleic acid binds to the target sequence (such as the time point at which the bioactive nucleic acid is substituted with the target sequence, and the time point at which target-specific release and binding occur) may be controlled in the presence of the target gene through control of the binding force between the bioactive peptide nucleic acid and the carrier peptide nucleic acid.

In the nucleic acid complex according to the present invention, the control of the time point of substitution (strand displacement) of the bioactive nucleic acid with the target gene and the time point of target-specific release and binding is made possible by the presence, number, and position of non-specific bases, universal bases and linkers of carrier nucleic acids for non-specific binding of the complex. The control is possible due to the combination of the factors described above and parallel or antiparallel binding, which is the complementary binding mode of the peptide complex.

As used herein, the term "blood-brain barrier" is used interchangeably with "BBB" and refers to a permeable barrier present within the blood because the blood circulates through brain tissue that closely regulates and strictly restricts the exchange between blood and brain tissue. Components of the blood-brain barrier include endothelial cells that form the deepest lining of all blood vessels, dense junctions between adjacent endothelial cells that are structurally correlated with the BBB, the basement membrane of endothelial cells, and an extended foot process of adjacent astrocytes covering almost all of the exposed outer surface of blood vessels. The BBB prevents most substances in the blood, including most large molecules therein, such as Ig, antibodies, complements, albumin, and drugs and small molecules, from entering brain tissue.

As used herein, the terms "disease" and "disorder" are used interchangeably and refers to any change in the state of the body or in some organs which impede and/or disrupt the performance of a function, cause symptoms such as discomfort and dysfunction, or lead to the death of people with a disease or the death of people who come into contact therewith.

In the present invention, the term "therapeutic composition" may be used interchangeably with "pharmaceutical composition", and refers to a composition that comprises, as an active ingredient, a nucleic acid complex comprising the bioactive nucleic acid and the carrier peptide nucleic acid bound to the nucleic acid according to the present invention, and may further comprise a therapeutic drug for treating a desired disease, bound to the nucleic acid complex.

The therapeutic composition of the present invention may be formulated in a form that can be delivered through the blood-brain barrier according to standard pharmaceutical practice. In addition to the active ingredient, these formulations may comprise additives such as carriers, excipients, adjuvants or diluents suitable for pharmaceutically acceptable formulations.

The term "physiologically acceptable" refers to a property of not impairing the biological activity and physical properties of a compound.

The term "carrier" is defined as a compound that facilitates the transport of the nucleic acid complex into cells or tissues. For example, dimethylsulfoxide (DMSO) is a commonly used carrier that facilitates the incorporation of many organic compounds into cells or tissues of an organism.

The term "diluent" is defined as a compound that stabilizes a biologically active form of a target compound and is diluted in water that dissolves the target compound. Salts dissolved in buffer solutions are used as diluents in the art. A commonly used buffer solution is phosphate-buffered saline because it mimics the salinity of human bodily fluids. Because buffer salts can control the pH of a solution at low concentrations, buffer diluents rarely alter the biological activity of compounds.

The substance comprising the nucleic acid complex in the present invention may be administered to a patient alone or as a pharmaceutical composition mixed with other active ingredients, or with suitable carriers or excipients, that is, in combination therapy.

The pharmaceutical composition suitable for use in the present invention comprises compositions comprising the active ingredients in an amount effective to achieve the intended purpose thereof. More specifically, a therapeutically effective amount means an amount of a compound effective to lengthen the survival of the subject to be treated, or to prevent, alleviate or relieve the symptoms of diseases. The determination of the therapeutically effective amount is possible by those skilled in the art, particularly in consideration of the detailed description provided herein.

The term "prevention" as used herein means any action of preventing the onset of a disease or inhibiting the progression thereof by administration of the therapeutic composition comprising the nucleic acid complex.

The term "alleviation" as used herein refers to any action of at least reducing the degree of parameters related to the condition to be treated, for example, the severity of symptoms, by administration of the therapeutic composition comprising the nucleic acid complex.

In addition, the term "treatment" as used herein refers to any action in which symptoms of a disease are alleviated or eliminated by administration of the therapeutic composition comprising the nucleic acid complex.

The composition comprising the nucleic acid complex according to the present invention may be applied in a pharmaceutically effective amount to treat glioblastoma or to inhibit (or alleviate) the symptoms of glioblastoma. The pharmaceutically effective amount may vary depending on various factors such as the type of glioblastoma, the age and weight of the patient, the characteristics and extent of symptoms, the type of current therapy, the number of treatments that are performed, and the application form and route, and can be easily determined by experts in the field. The composition of the present invention may be applied simultaneously or sequentially in combination with the pharmacological or physiological components described above, and may be applied sequentially or simultaneously in combination with additional conventional therapeutic agents. Administration may be performed in one or multiple applications.

As used herein, the term "subject" refers to a mammal, preferably a human that suffers from or is at risk of a condition or disease that can be alleviated, suppressed or treated by administering the nucleic acid complex according to the present invention thereto.

In addition, the amount of the compound of the present invention that is administered to the human body may vary depending on the age, weight and gender of the patient, the administration form, the health status, and the severity of disease, and is generally 0.001 to 1,000 mg/day, preferably 0.01 to 500 mg/day, based on an adult patient weighing 70 kg, and may be administered once a day or in multiple doses (in a portionwise manner) several times a day at regular time intervals according to the prescription of doctors or pharmacists.

The toxicity and therapeutic efficiency of the compositions comprising the nucleic acid complex described herein are, for example, estimated through standard pharmaceutical procedures in cell culture or laboratory animals to determine the LD50 (lethal dose for 50% of the population), ED50 (dose providing a therapeutic effect on 50% of the population) and IC50 (dose providing therapeutic and inhibitory effects on 50% of the population). The ratio of toxicity to therapeutic effect for a dose is called the therapeutic index, and may be expressed as the ratio of LD50 to ED50 (or IC50). Compounds having a high therapeutic index are preferred. The data obtained from these cell culture assays may be used to estimate the range of dose for human applications. The amount (dosage) of such a compound that is administered is preferably within a range of a circulating concentration including ED50 (or IC50) with little or no toxicity.

As used herein, the term "administration" refers to an act of introducing the pharmaceutical composition of the present invention into a subject by any suitable method, and the administration may be performed through any of various routes, either oral or parenteral, as long as it enables the composition to reach the target tissue.

The pharmaceutical composition of the present invention may be administered in a pharmaceutically effective amount. The term "pharmaceutically effective amount" used herein means a sufficient amount used to treat or prevent a disease at a reasonable benefit/risk ratio applicable to medical treatment or prevention. The effective amount is determined depending on factors including the severity of the disease, the activity of the drug, the age, weight, health and gender of the patient, the sensitivity of the patient to the drug, the time of administration, the route of administration, and the rate of excretion and treatment period of the composition of the present invention used, drugs used in combination with or concurrently with the composition of the present invention, and other factors well known in the pharmaceutical field.

The pharmaceutical composition of the present invention may be administered as a single therapeutic agent or in combination with other therapeutic agents, either sequentially or simultaneously. The pharmaceutical composition of the present invention may be administered in single or multiple doses. Taking into consideration these factors, it is important to administer the composition in the minimum amount sufficient to achieve maximum efficacy without side effects.

In addition, the dosage (administered amount) of the pharmaceutical composition according to the present invention may be determined by those skilled in the art in consideration of the purpose of use, the severity of disease, the patient's age, weight, gender, and history, or the substances used as active ingredients. For example, the pharmaceutical composition may be administered to an adult in a daily dose of 10 mg/kg to 100 mg/kg, more preferably 1 mg/kg to 30 mg/kg. The frequency of administration of the composition of the present invention is not particularly limited, and the composition may be administered one to three times a day, or may be divided into multiple doses and administered throughout the day.

In another aspect, the present invention is directed to a method of preventing or treating glioblastoma comprising administering, to a subject, a nucleic acid complex in which a bioactive peptide nucleic acid having a sequence capable of binding to a TGF-$\beta$2 gene complementarily binds to a carrier peptide nucleic acid, and/or a composition comprising the nucleic acid complex.

In another aspect, the present invention is directed to the use of a nucleic acid complex in which a bioactive peptide nucleic acid having a sequence capable of binding to a TGF-$\beta$2 gene complementarily binds to a carrier peptide nucleic acid, and/or a composition comprising the nucleic acid complex for the manufacture of a medicament for preventing or treating of glioblastoma.

In another aspect, the present invention is directed to the use of a nucleic acid complex in which a bioactive peptide nucleic acid having a sequence capable of binding to a TGF-$\beta$2 gene complementarily binds to a carrier peptide nucleic acid, and/or a composition comprising the nucleic acid complex for the prevention or treatment of glioblastoma.

**[Example]**

Hereinafter, the present invention will be described in more detail with reference to the following examples. However, it will be obvious to those skilled in the art that the following examples are provided only for illustration of the present invention and should not be construed as limiting the scope of the present invention based on the subject matter of the present invention.

**Example 1: Bioactive peptide nucleic acid and carrier peptide nucleic acid, and preparation of complex using the same**

TGF-$\beta$2 was used as a target gene to verify the effect on glioblastoma of the nucleic acid complex of the present invention. An antisense peptide nucleic acid (antisense PNA) was used as the bioactive nucleic acid for TGF-$\beta$2 to determine the therapeutic effect on glioblastoma.

The bioactive nucleic acid (antisense PNA) used to determine the therapeutic effect on glioblastoma has sequences represented by SEQ ID NOS: 1 and 2 and the bioactive nucleic acid (antisense PNA) used as a control of the present invention has a sequence represented by SEQ ID NO: 3.

The peptide-nucleic-acid-based bioactive nucleic acids used in this example, represented by SEQ ID NOS: 2 and 3, have a 5' end bound with GLFDIIKKIAESF, which is a peptide for facilitating the endosomal escape, and the nucleic acid sequence represented by SEQ ID NO: 1 was synthesized without the peptide to promote the endosomes escape. All carrier peptide nucleic acids used in this example, excluding the sequence represented by SEQ ID NO: 9 have a 5' or 3' end bound with peptides to promote endosomal escape to thereof and have sequences represented by SEQ ID NOS: 4 to 8. Base sequences, monomer modifications and structures are shown in Table 1 below. All peptide nucleic acids used in the present invention were synthesized by HPLC purification at Panagene (Korea) (Table 1).

[Table 1]

| Sequences of bioactive nucleic acids and carrier peptide nucleic acids to verify therapeutic effect for glioblastoma | | | | |
|---|---|---|---|---|
| Item | SEQ ID NO: | Endosomal escape peptide | Base sequence | Monomer modification |
| Bioactive nucleic acid | 1 | - | 5'-AAC$^{(-)}$TG$^{(+)}$GT$^{(-)}$CCA$^{(+)}$TAT$^{(-)}$CG-O-K-3' | -+-+- |
| | 2 | GLFDIIKKIAESF | 5'-GLFDIIKKIAESF-O-AAC$^{(-)}$TG$^{(+)}$GT$^{(-)}$CCA$^{(+)}$TAT$^{(-)}$CG-O-K-3' | -+-+- |
| | 3 | GLFDIIKKIAESF | 5'-GLFDIIKKIAESF-O-T$^{(-)}$ATA$^{(+)}$TC$^{(-)}$GG$^{(+)}$TCTA$^{(-)}$AGC-O-K-3' | -+-+- |
| Carrier peptide nucleic acid | 4 | Histidine (10) | 5'-Histidine(10)-O-TTG$^{(+)}$ACCA$^{(+)}$GGTATA$^{(+)}$GC-O-K-3' | +++ |
| | 5 | Histidine(10) | 5'-K-O-CG$^{(+)}$ATATGG$^{(+)}$ACCA$^{(+)}$GTT-O-Histidine(10)-3 | +++ |
| | 6 | Histidine(10) | 5'-Histidine(10)-O-AT$^{(+)}$AG$^{(+)}$C-O-K-3' | ++ |
| | 7 | Histidine(10) | 5'-K-O-CG$^{(+)}$AT$^{(+)}$A-O-Histidine(10)-3' | ++ |
| | 8 | Histidine(10) | 5'-Histidine(10)-O-ATA$^{(+)}$TAGCCA$^{(+)}$GATT$^{(+)}$CG-O-K-3' | +++ |
| | 9 | - | 5'-K-O-CG$^{(+)}$ATATGG$^{(+)}$ACCA$^{(+)}$GTT-3' | +++ |

Table 1 above shows the sequence information of the bioactive peptide nucleic acid and carrier peptide nucleic acid used to determine the anticancer effect for glioblastoma targeting TGF-β2 and the sequence information of bioactive peptide nucleic acid and carrier peptide nucleic acid used as a control.

In order to impart electrical properties, modification of the monomer was performed to produce a modified peptide nucleic acid backbone designed such that the peptide nucleic acid backbone comprises lysine (Lys, K$^{(+)}$) for a positive electrical charge and the modified peptide nucleic acid backbone comprises glutamic acid (Glu, E$^{(-)}$) for a negative electrical charge.

The respective combinations of bioactive nucleic acids and carrier peptide nucleic acids were hybridized in the presence of DMSO to produce a complex comprising the bioactive nucleic acid and the carrier peptide nucleic acid.

**Example 2: Analysis of therapeutic effect for glioblastoma using nucleic acid complexes**

The anticancer effect for glioblastoma was analyzed using the nucleic acid complex comprising the carrier peptide nucleic acid and the bioactive peptide nucleic acid targeting TGF-β2 prepared to have the structure of the following Table 2 according to Example 1.

[Table 2]

| Combination of nucleic acid complexes for gene expression analysis in human glioblastoma cell lines | |
|---|---|
| Item | Nucleic acid complex |
| 1 | SEQ ID NOS: 3 and 8 (control) |

(continued)

| Combination of nucleic acid complexes for gene expression analysis in human glioblastoma cell lines | |
| --- | --- |
| Item | Nucleic acid complex |
| 2 | SEQ ID NOS: 2 and 4 |
| 3 | SEQ ID NOS: 2 and 5 |
| 4 | SEQ ID NOS: 2 and 6 |
| 5 | SEQ ID NOS: 2 and 7 |
| 6 | SEQ ID NOS: 1 and 9 |

Example 2-1: Cell culture

Human glioblastoma cell (U87MG, ATCC No. HTB-14) obtained from ATCC (American Type Culture Collection, USA) was incubated at 37°C in the presence of 5% (v/v) $CO_2$ in RPMI-1640 culture medium (Wellgene, Korea) supplemented with 10% (v/v) fetal bovine serum, 100 units/ml of penicillin, and 100 pg/ml of streptomycin.

Example 2-2: Analysis of gene expression in cells treated with peptide nucleic acid complexes

Human glioblastoma cells were seeded at $1 \times 10^5$ in a 96-well plate, cultured for 24 hours, and tested under the experimental conditions in Example 2-1, the cell lines were treated with 500 nM of a complex comprising bioactive peptide nucleic acid and carrier peptide nucleic acid, incubated for 24, 48, 72, 96, and 120 hours, and 30 $\mu$L of RIPA buffer was added to each well to obtain a protein lysate.

The protein was assayed from the protein lysate using a BCA assay kit (Thermo Fisher, USA), 30 $\mu$g of protein was separated by size through electrophoresis, and the protein was transferred to a PVDF membrane, followed by treatment with primary antibodies, TGF-β2 (Santa Cruz Biotechnology, USA) and p-Smad3 (Cell signaling technology, USA) at a ratio of 1:1000 and allowing to stand at 4°C for one day.

The product was washed with 1XT BS-T, treated with, as secondary antibodies, goat anti-rabbit (Cell Signaling Technology, USA) and anti-mouse (Santa Cruz Biotechnology, USA) at a ratio of 1:2,000, and allowed to stand at room temperature for 1 hour. The result was treated with Supersignal™ West Femto Maximum Sensitivity Substrate (Thermo Fisher, USA), and the efficiency of suppression of target gene expression was analyzed using an Imager 600 (Amersham, Germany).

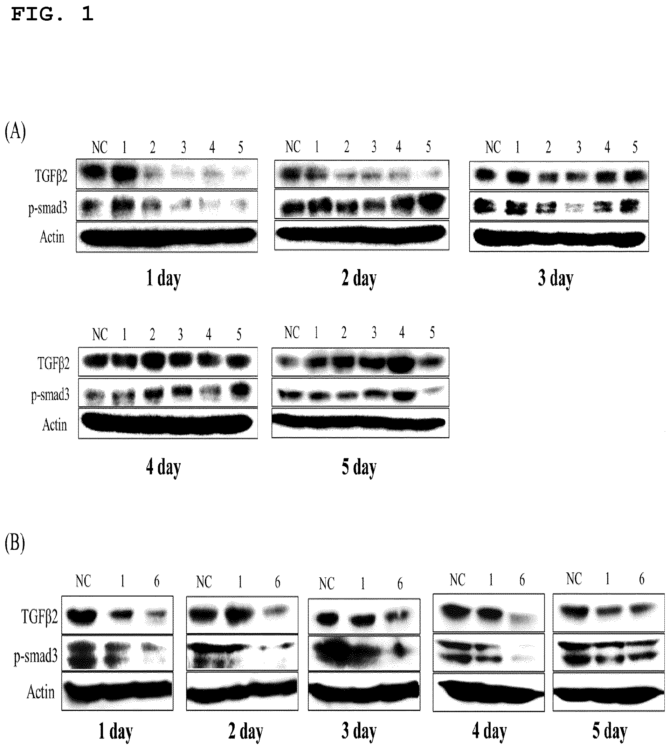

As a result, as can be seen from FIG. 1, the groups treated with the nucleic acid complex of combination of SEQ ID NOS: 2 and 5 and the nucleic acid complex of combination of SEQ ID NOS: 2 and 7 inhibited expression of the target gene and the downstream gene for up to 72 hours the most (FIG. 1A).

In addition, the efficiency of inhibiting the expression of the target gene of the nucleic acid complexes (SEQ ID NOS: 1 and 9, Combination 6) of the sequence not having the peptide facilitating the endosomal escape function, compared to the nucleic acid complexes of SEQ ID NOS: 2 and 5 (Combination 3), was determined. The result showed that the nucleic acid complex (SEQ ID NOS: 1 and 9, combination 6) inhibited the expression of the target gene (TGF-β2) and downstream gene (p-Smad3) for up to 96 hours, and inhibited the target gene for longer than the nucleic acid complex combination (SEQ ID NOS: 2 and 5, combination 3) having peptide facilitating endosomal escape function (FIG. 1B).

Example 2-3: Analysis of cell migration of cells treated with peptide nucleic acid complex

A cell culture insert (BD, USA) was fixed in a 24-well plate and then human glioblastoma cells are seeded at $1 \times 10^4$ inside the insert using a medium containing no fetal bovine serum, and a medium containing 10% fetal bovine serum was added to a 24-well plate in a part under the insert, followed by incubating for 24 hours, conducting the experiment under the conditions of Example 2-1, and treating the result with 500 nM of the complex comprising the bioactive peptide nucleic acid and the carrier peptide nucleic acid. The cells were cultured for 24, 48, and 72 hours, were fixed with 4% PFA (paraformaldehyde) for 10 minutes, and stained with 0.1% crystal violet solution for 30 minutes, the remaining cells inside the insert were removed with a cotton swab and a staining solution was washed with water. The insert membrane was dried for about a day, and the number of migrating cells was observed under a microscope.

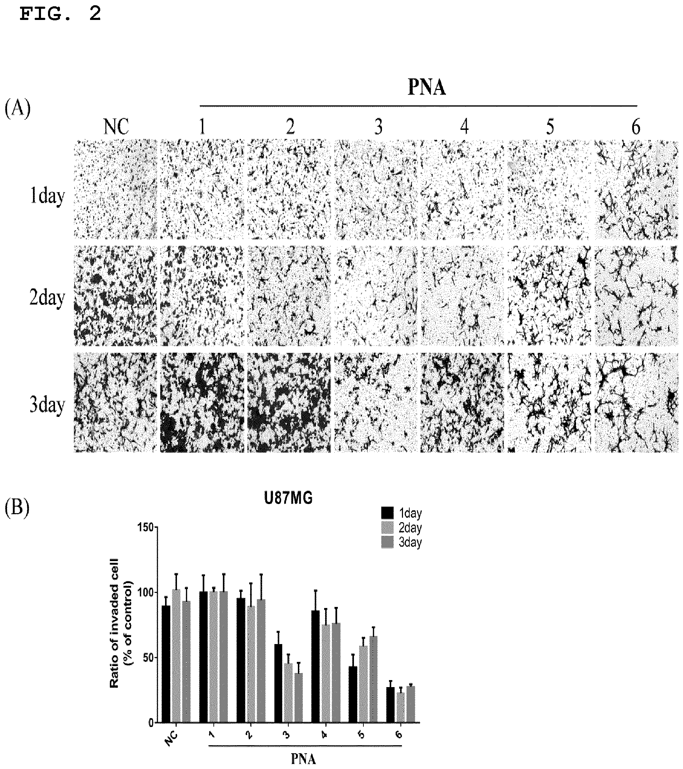

As a result, as can be seen from FIG. 2, the cell migration efficiency was most inhibited in the combination of nucleic acid complex of SEQ ID NOS: 2 and 5, and this inhibitory effect continued for up to 72 hours (FIG. 2A).

In addition, the cell migration inhibition efficiency of the nucleic acid complex (SEQ ID NOS: 1 and 9, combination 6) of the sequence having no peptide facilitating the endosomal escape function was observed in the nucleic acid complex of SEQ ID NOS: 2 and 5 (combination 3). The result showed that the group treated with the nucleic acid complex having no peptide also inhibited cell migration and exhibited higher inhibition efficiency than that of the group treated with the nucleic acid complex having the peptide (combination 3) (FIG. 2A).

The migrating cells were quantitatively confirmed using the ImageJ program. The result showed that the group treated with the nucleic acid complex combination of SEQ ID NOS: 2 and 5 (combination 3) decreased by about 50% or more compared to the control group up to 72 hours and the group treated with the nucleic acid complex having no peptide (combination 6) reduced cell migration by more than 70% from 24 hours, compared to the control group, and inhibited cells faster than the nucleic acid complex with peptides (SEQ ID NOS: 2 and 5, combination 3) facilitating endosomal escape (FIG. 2B).

**Example 3: Analysis of glioblastoma phenotype effect in orthotopic mouse model**

To verify the efficacy in an orthotopic animal model, 7-week-old female nude mice (Orient Bio, Korea) were used. To construct a tumor model, mice were anesthetized with Avertin (5 mg/kg), fixed in a brain stereotaxic device for small animals (Stereotaxic instruments, Stoelting Co, USA), and $3\times10^5$ U87MG cells were injected using a Hamilton syringe. The injection coordinates are AP +0.5 mm, ML +2.0 mm, and DV -3 mm. After the tumor was allowed to grow for 10 days, the nucleic acid complex was administered through the tail vein twice a week, and as a positive control, galunisertib (MedChemexpress, USA), a TGF-β receptor inhibitor, was orally administered daily at a dose of 75 mg/kg. During the administration period, body weight was measured twice a week, and all animals were euthanized after 6 administrations over 3 weeks.

In this example, nucleic acid combinations whose effects were verified in Example 2 were selected and histological findings and changes in gene expression of TGF-β2 were analyzed in an orthotopic animal model of U87MG glioblastoma cells. The nucleic acid complex combinations used were shown in Table 3.

[Table 3]

| Combinations of nucleic acid complexes for analysis of tumor treatment effect in glioblastoma orthotopic animal model | |
|---|---|
| Combination | Nucleic Acid Complex |
| 3 | SEQ ID NOS: 2 and 5 |
| 6 | SEQ ID NOS: 1 and 9 |

Example 3-1: Phenotypic analysis in glioblastoma orthotopic transplant animal model using H&E staining

The experiment was conducted under the conditions of Example 3. At the end of the final experiment, mouse brain tissue was biopsied, fixed in a 4% formalin solution for one day, immersed in a 30% sugar solution, dehydrated for 3 days, and then embedded in an OCT solution. The embedded tissue was stored at -20°C and frozen, and then the tissue was sectioned at 20 um in a cryostat (Cryostat, Leica, USA) and mounted on a slide glass. The mounted tissue was stained with Hematoxylin:Eosin staining solution for a certain period of time, washed with 1X PBS, and then size change of the tumor was analyzed under a microscope.

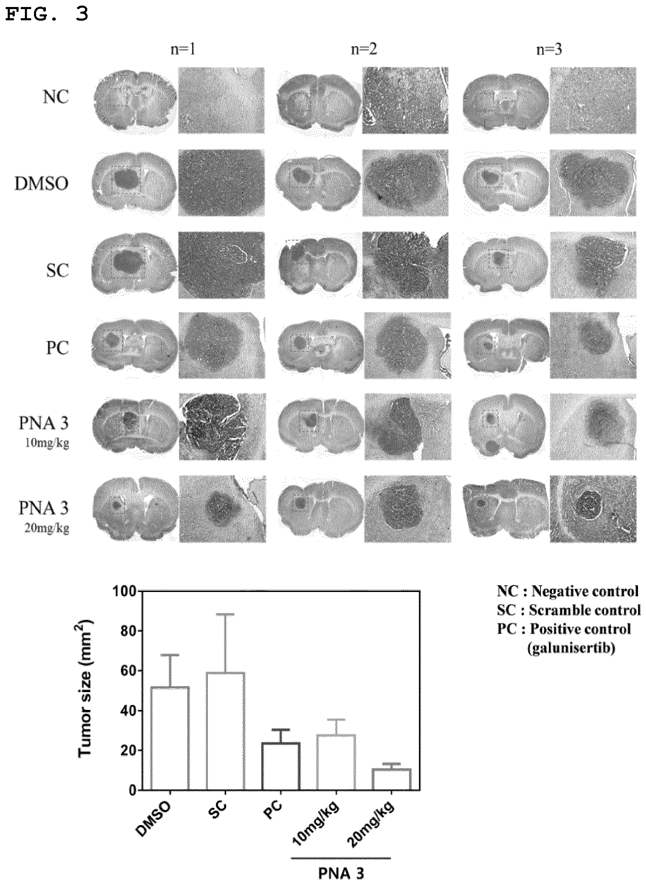

As a result, as can be seen from FIG. 3, the group treated with the nucleic acid complex combination of SEQ ID NOS: 2 and 5 (combination 3) significantly reduced the size of the tumor, compared to the induction group (DMSO) implanted with the tumor, and the high concentration administration group (PNA 3, 20 mg/kg) reduced the size of the tumor more than that of the positive control (PC) (FIG. 3). The size of the tumor was quantitatively expressed by measuring the horizontal and vertical lengths of the tumor using the imageJ program and then calculating the area.

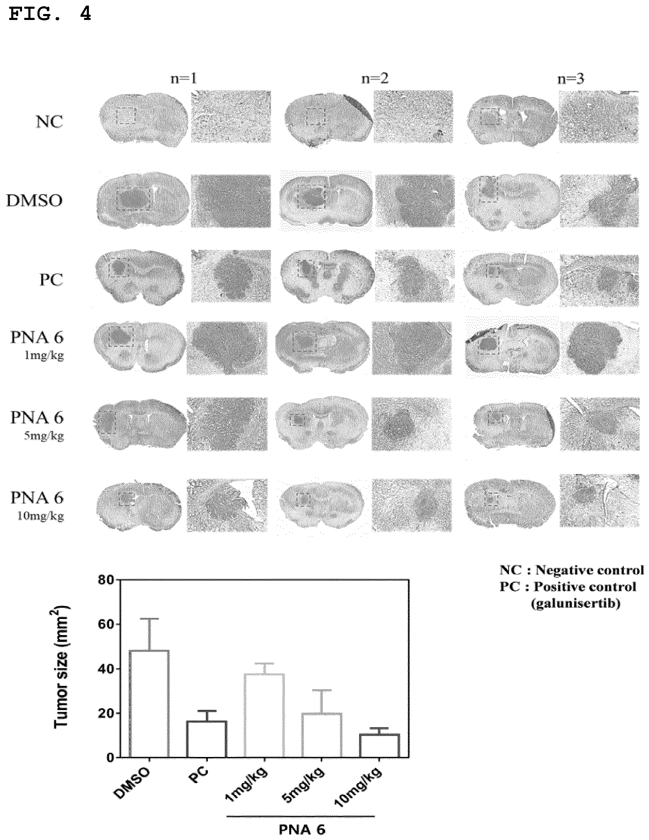

In addition, the histological tumor size change of the nucleic acid complex (SEQ ID NOS: 1 and 9, combination 6) with the sequence having no peptide facilitating the endosomal escape function in the nucleic acid complex of SEQ ID NOS: 2 and 5 (combination 3) was confirmed. The result showed that the group treated with the nucleic acid complex having no peptide reduced the size of the tumor as the treatment concentration increased, compared to the tumor induction control (DMSO). Also, the area of the tumor was measured and quantified using the ImageJ program. The result showed that the group treated with the nucleic acid complex decreased the size of the tumor in a concentration-dependent manner (FIG. 4). In particular, the 10 mg/kg administration group (PNA 6, 10 mg/kg), which reduced the dose by half, compared to the nucleic acid complex with peptide (combination 3), significantly reduced the size of the tumor and exhibited similar inhibitory efficiency to the high concentration treatment group of the nucleic acid complex with peptide (combination 3, 20 mg/kg) (FIGS. 3 and 4).

Example 3-2: Analysis of gene expression by Western blot assay

RIPA buffer was added at 200 μL to the biopsied mouse tumor tissue after the experiment under the conditions of Example 3 to obtain a protein lysate. The protein was assayed from the protein lysate using a BCA assay kit (Thermo Fisher, USA), 30 μg of protein was separated by size through electrophoresis, and the protein was transferred to a PVDF membrane, treated at a ratio of 1:1000 with primary antibodies, namely, TGF-β2 (Santa Cruz Biotechnology, US), p-Smad3 (Cell signaling technology, US) and allowed to stand at 4°C for one day.

The result was washed with 1X TBS-T, treated with secondary antibodies, namely, Goat Anti-Rabbit (Cell Signaling Technology, USA) and Anti-Mouse (Santa Cruz Biotechnology, USA) at a ratio of 1:2000 and allowed to stand at room temperature for 1 hour. The result was treated with Supersignal™ West Femto Maximum Sensitivity Substrate (Thermo Fisher, USA) and efficiency of suppression of target gene expression was analyzed in mouse brain tissue using an Imager 600 (Amersham, Germany).

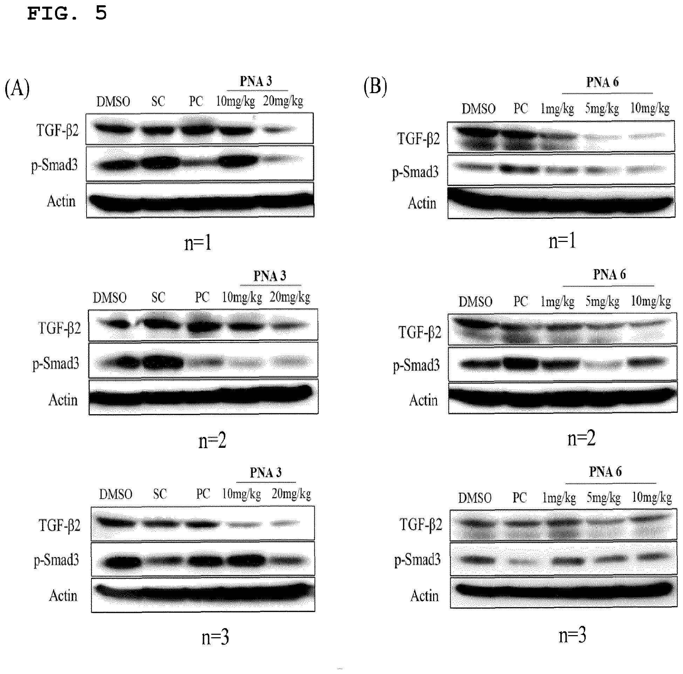

As a result, as can be seen from FIG. 5, the group treated with the nucleic acid complex of SEQ ID NOS: 2 and 5 (combination 3) inhibited expression of the targeting gene TGF-β2 and expression of the downstream gene p-Smad3, compared to tumor-inducing groups (FIG. 2A), and the high-concentration treatment group (PNA 3, 20 mg/kg) inhibited the expression of target genes and downstream genes for n=3 (FIG. 5A).

In addition, the group treated with the nucleic acid complex (SEQ ID NOS: 1 and 9, combination 6) of the sequence having no peptide facilitating the endosomal escape function, in the nucleic acid complex of SEQ ID NOS: 2 and 5 (combination 3), inhibited the target gene compared to the tumor-inducing group. In particular, the two high concentration treatment groups (PNA 6, 5 mg/kg and 10 mg/kg) inhibited the expression of target genes and downstream genes for n = 3, compared to other tumor induction groups (FIG. 5B) .

**Industrial Applicability**

The nucleic acid complex according to the present invention has excellent cell penetration and intracellular activity and efficiently inhibits expression of TGF-β2 gene and downstream genes thereof, thus being useful for prevention or treatment of glioblastoma.

Although specific configurations of the present invention have been described in detail, those skilled in the art will appreciate that this description is provided to set forth preferred embodiments for illustrative purposes, and should not be construed as limiting the scope of the present invention. Therefore, the substantial scope of the present invention is defined by the accompanying claims and equivalents thereto.

**Sequence List Free Text**

An electronic file is attached.

**Claims**

1. A pharmaceutical composition for preventing, ameliorating or treating glioblastoma comprising, as an active ingredient, a cell-permeable nucleic acid complex in which a carrier peptide nucleic acid complementarily binds to a bioactive nucleic acid targeting a TGF-β2 gene.

2. The pharmaceutical composition according to claim 1, wherein the bioactive peptide nucleic acid comprises a sequence represented by SEQ ID NO: 1.

3. The pharmaceutical composition according to claim 1, wherein the carrier peptide nucleic acid comprises a sequence selected from the group consisting of sequences represented by SEQ ID NOS: 4 to 7 and 9.

4. The pharmaceutical composition according to claim 1, wherein the nucleic acid complex is selected from the group consisting of:

   (i) a nucleic acid complex comprising a bioactive nucleic acid having a sequence represented by SEQ ID NO: 1 and a carrier peptide nucleic acid having a sequence represented by SEQ ID NO: 4;
   (ii) a nucleic acid complex comprising a bioactive nucleic acid having a sequence represented by SEQ ID NO: 1 and a carrier peptide nucleic acid having a sequence represented by SEQ ID NO: 5;
   (iii) a nucleic acid complex comprising a bioactive nucleic acid having a sequence represented by SEQ ID NO: 1 and a carrier peptide nucleic acid having a sequence represented by SEQ ID NO: 6;

(iv) a nucleic acid complex comprising a bioactive nucleic acid having a sequence represented by SEQ ID NO: 1 and a carrier peptide nucleic acid having a sequence represented by SEQ ID NO: 7; and

(v) a nucleic acid complex comprising a bioactive nucleic acid having a sequence represented by SEQ ID NO: 1 and a carrier peptide nucleic acid having a sequence represented by SEQ ID NO: 9.

5. The pharmaceutical composition according to claim 1, wherein the bioactive nucleic acid or the carrier peptide nucleic acid comprises a material for facilitating endosomal escape that is additionally bound to a 5'-end or a 3'-end of each nucleic acid.

6. The pharmaceutical composition according to claim 5, wherein the material for facilitating endosomal escape is at least one selected from the group consisting of peptides, lipid nanoparticles, polyplex nanoparticles, polymer nanospheres, inorganic nanoparticles, cationic lipid-based nanoparticles, cationic polymers, and pH-sensitive polymers.

7. The pharmaceutical composition according to claim 6, wherein the peptide for facilitating endosomal escape is GLFDIIKKIAESF (SEQ ID NO: 10) or histidine(10).

8. The pharmaceutical composition according to claim 1, wherein the bioactive nucleic acid has a net negative charge or neutral.

9. The pharmaceutical composition according to claim 1, wherein the carrier peptide nucleic acid has a net positive charge.

10. The pharmaceutical composition according to claim 1, wherein the nucleic acid complex has a net positive charge.

11. The pharmaceutical composition according to claim 1, wherein the nucleic acid complex is capable of penetrating a blood-brain barrier.

FIG. 1

(A)

1 day    2 day    3 day

4 day    5 day

(B)

1 day    2 day    3 day    4 day    5 day

FIG. 2

FIG. 3

NC : Negative control
SC : Scramble control
PC : Positive control
(galunisertib)

FIG. 4

NC : Negative control
PC : Positive control
(galunisertib)

FIG. 5

(A)

n=1

n=2

n=3

(B)

n=1

n=2

n=3

## INTERNATIONAL SEARCH REPORT

| International application No. |
| --- |
| **PCT/KR2021/018965** |

**A. CLASSIFICATION OF SUBJECT MATTER**

**A61K 48/00**(2006.01)i; **A61K 47/54**(2017.01)i; **A61K 47/64**(2017.01)i; **A61K 31/7088**(2006.01)i; **A61P 35/00**(2006.01)i; **C12N 15/113**(2010.01)i

According to International Patent Classification (IPC) or to both national classification and IPC

**B. FIELDS SEARCHED**

Minimum documentation searched (classification system followed by classification symbols)

A61K 48/00(2006.01); A61K 31/7125(2006.01); A61K 47/54(2017.01); A61K 47/64(2017.01); C12N 15/11(2006.01); C12Q 1/68(2006.01); C12Q 1/6883(2018.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: TGF-β2, 생활성 펩티드 핵산(bioactive peptide nucleic acid), 캐리어 펩티드 핵산 (carrier peptide nucleic acid), 교모세포종(glioblastoma), 엔도좀 탈출(endosome escape), 핵산 복합체(nucleic acid complex)

**C. DOCUMENTS CONSIDERED TO BE RELEVANT**

| Category* | Citation of document, with indication, where appropriate, of the relevant passages | Relevant to claim No. |
| --- | --- | --- |
| Y | KR 10-2019-0096148 A (SEASUN THERAPEUTICS) 19 August 2019 (2019-08-19) See claims 1-4, 9, 25 and 26. | 1,5-11 |
| A | | 2-4 |
| Y | EP 1008649 A2 (BIOGNOSTIK GESELLSCHAFT FUR BIOMOLEKULARE DIAGNOSTIK MBH) 14 June 2000 (2000-06-14) See claims 1 and 5-7. | 1,5-11 |
| Y | US 2004-0034043 A1 (KATZHENDLER, J. et al.) 19 February 2004 (2004-02-19) See claim 31; and paragraph [0064]. | 11 |
| Y | KR 10-2020-0090419 A (SEASUN THERAPEUTICS) 29 July 2020 (2020-07-29) See claims 1 and 4-8. | 1,5-11 |

☑ Further documents are listed in the continuation of Box C.   ☑ See patent family annex.

| | | |
| --- | --- | --- |
| * | Special categories of cited documents: | |
| "A" | document defining the general state of the art which is not considered to be of particular relevance | "T" later document published after the international filing date or priority date and not in conflict with the application but cited to understand the principle or theory underlying the invention |
| "D" | document cited by the applicant in the international application | "X" document of particular relevance; the claimed invention cannot be considered novel or cannot be considered to involve an inventive step when the document is taken alone |
| "E" | earlier application or patent but published on or after the international filing date | |
| "L" | document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" | document referring to an oral disclosure, use, exhibition or other means | "&" document member of the same patent family |
| "P" | document published prior to the international filing date but later than the priority date claimed | |

| Date of the actual completion of the international search | Date of mailing of the international search report |
| --- | --- |
| **23 March 2022** | **23 March 2022** |

| Name and mailing address of the ISA/KR | Authorized officer |
| --- | --- |
| **Korean Intellectual Property Office** **Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**

| | International application No. |
|---|---|
| | **PCT/KR2021/018965** |

**C.    DOCUMENTS CONSIDERED TO BE RELEVANT**

| Category* | Citation of document, with indication, where appropriate, of the relevant passages | Relevant to claim No. |
|---|---|---|
| Y | KR 10-2018-0018405 A (SEASUN BIOMATERIALS) 21 February 2018 (2018-02-21)<br>      See claims 1 and 22-24. | 1,8-11 |

Form PCT/ISA/210 (second sheet) (July 2019)

## INTERNATIONAL SEARCH REPORT

International application No.

**PCT/KR2021/018965**

| Box No. I | Nucleotide and/or amino acid sequence(s) (Continuation of item 1.c of the first sheet) |
|---|---|

1. With regard to any nucleotide and/or amino acid sequence disclosed in the international application, the international search was carried out on the basis of a sequence listing:

   a. ☑ forming part of the international application as filed:

      ☑ in the form of an Annex C/ST.25 text file.

      ☐ on paper or in the form of an image file.

   b. ☐ furnished together with the international application under PCT Rule 13*ter*.1(a) for the purposes of international search only in the form of an Annex C/ST.25 text file.

   c. ☐ furnished subsequent to the international filing date for the purposes of international search only:

      ☐ in the form of an Annex C/ST.25 text file (Rule 13*ter*.1(a)).

      ☐ on paper or in the form of an image file (Rule 13*ter*.1(b) and Administrative Instructions, Section 713).

2. ☐ In addition, in the case that more than one version or copy of a sequence listing has been filed or furnished, the required statements that the information in the subsequent or additional copies is identical to that forming part of the application as filed or does not go beyond the application as filed, as appropriate, were furnished.

3. Additional comments:

Form PCT/ISA/210 (continuation of first sheet) (July 2019)

<div style="text-align: center">

**INTERNATIONAL SEARCH REPORT**
**Information on patent family members**

</div>

International application No.

**PCT/KR2021/018965**

| Patent document cited in search report | | | Publication date (day/month/year) | Patent family member(s) | | | Publication date (day/month/year) |
|---|---|---|---|---|---|---|---|
| KR | 10-2019-0096148 | A | 19 August 2019 | AU | 2019-219472 | A1 | 10 September 2020 |
| | | | | CA | 3090754 | A1 | 15 August 2019 |
| | | | | CN | 112135907 | A | 25 December 2020 |
| | | | | EP | 3750995 | A1 | 16 December 2020 |
| | | | | JP | 2021-515589 | A | 24 June 2021 |
| | | | | KR | 10-2262260 | B1 | 09 June 2021 |
| | | | | US | 2021-0171953 | A1 | 10 June 2021 |
| | | | | WO | 2019-156365 | A1 | 15 August 2019 |
| EP | 1008649 | A2 | 14 June 2000 | AT | 211762 | T | 15 January 2002 |
| | | | | AT | 235549 | T | 15 April 2003 |
| | | | | AU | 6794594 | A | 21 November 1994 |
| | | | | EP | 0695354 | A1 | 07 February 1996 |
| | | | | EP | 0695354 | B1 | 09 January 2002 |
| | | | | EP | 1008649 | A3 | 12 July 2000 |
| | | | | EP | 1008649 | B1 | 26 March 2003 |
| | | | | EP | 1160319 | A2 | 05 December 2001 |
| | | | | EP | 1160319 | A3 | 06 March 2002 |
| | | | | JP | 2004-024264 | A | 29 January 2004 |
| | | | | JP | 3555952 | B2 | 18 August 2004 |
| | | | | JP | 3600831 | B2 | 15 December 2004 |
| | | | | JP | 8509370 | A | 08 October 1996 |
| | | | | US | 2003-0040499 | A1 | 27 February 2003 |
| | | | | US | 2008-0214483 | A1 | 04 September 2008 |
| | | | | US | 6455689 | B1 | 24 September 2002 |
| | | | | US | 7667027 | B2 | 23 February 2010 |
| | | | | WO | 94-25588 | A2 | 10 November 1994 |
| | | | | WO | 94-25588 | A3 | 22 December 1994 |
| US | 2004-0034043 | A1 | 19 February 2004 | AU | 2001-67788 | A1 | 02 January 2002 |
| | | | | AU | 2001-67788 | B2 | 04 May 2006 |
| | | | | CA | 2413556 | A1 | 27 December 2001 |
| | | | | EP | 1292606 | A2 | 19 March 2003 |
| | | | | IL | 153525 | A | 06 July 2003 |
| | | | | JP | 2004-520261 | A | 08 July 2004 |
| | | | | US | 7671036 | B2 | 02 March 2010 |
| | | | | WO | 01-098522 | A3 | 08 August 2002 |
| | | | | WO | 01-98522 | A2 | 27 December 2001 |
| KR | 10-2020-0090419 | A | 29 July 2020 | KR | 10-2156324 | B1 | 15 September 2020 |
| KR | 10-2018-0018405 | A | 21 February 2018 | AU | 2017-310146 | A1 | 28 March 2019 |
| | | | | AU | 2017-310146 | B2 | 17 December 2020 |
| | | | | CA | 3033474 | A1 | 15 February 2018 |
| | | | | CN | 109804070 | A | 24 May 2019 |
| | | | | EP | 3498844 | A1 | 19 June 2019 |
| | | | | JP | 2019-526624 | A | 19 September 2019 |
| | | | | JP | 6818889 | B2 | 20 January 2021 |
| | | | | KR | 10-1963885 | B1 | 01 April 2019 |
| | | | | KR | 10-2018-0100523 | A | 11 September 2018 |
| | | | | US | 10745446 | B2 | 18 August 2020 |
| | | | | US | 2019-0185519 | A1 | 20 June 2019 |
| | | | | WO | 2018-030789 | A1 | 15 February 2018 |

Form PCT/ISA/210 (patent family annex) (July 2019)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2012061120 A
- WO 2012104822 A
- KR 2017008636 W
- KR 1020190128465

**Non-patent literature cited in the description**

- **JOANNA L. BIRCH et al.** *Cellular Signaling,* 2020, vol. 72, 109638
- **ROY LO ; POIRIER MB ; FORTIN D.** *Int. J. Mol. Sci.,* 2018, vol. 19 (4), 1113
- **KOLE R. et al.** *Nature Rev. Drug Discov.,* 2012, vol. 11, 125
- **WILSON C. et al.** *Curr. Opin. Chem. Bio.,* 2006, vol. 10, 607
- **D. W. PACK ; A. S. HOFFMAN ; S. PUN ; P. S. STAYTON.** Design and development of polymers for gene delivery. *Nat. Rev. Drug. Discov.,* 2005, vol. 4, 581-593